(19) Europäisches Patentamt
European Patent Office
Office européen des brevets

(11) Veröffentlichungsnummer : **0 446 783 B1**

# (12) EUROPÄISCHE PATENTSCHRIFT

(45) Veröffentlichungstag der Patentschrift : **27.04.94 Patentblatt 94/17**

(51) Int. Cl.[5] : **C07C 213/02,** C07D 295/02

(21) Anmeldenummer : **91103485.8**

(22) Anmeldetag : **07.03.91**

## (54) Verfahren zur Herstellung von N-aryl-substituierten 2-Aminoalkyl-2-hydroxy-alkylaminen und N-arylsubstituierten Piperazinen.

(30) Priorität : **15.03.90 DE 4008322**

(43) Veröffentlichungstag der Anmeldung : **18.09.91 Patentblatt 91/38**

(45) Bekanntmachung des Hinweises auf die Patenterteilung : **27.04.94 Patentblatt 94/17**

(84) Benannte Vertragsstaaten : **BE CH DE GB IT LI**

(56) Entgegenhaltungen : **EP-A- 0 235 651**

(56) Entgegenhaltungen :
**DE-B- 1 186 069**
**FR-A- 1 186 341**
**FR-A- 1 575 059**
**FR-A- 2 313 369**
**GB-A- 902 570**

(73) Patentinhaber : **BASF Aktiengesellschaft**
**Carl-Bosch-Strasse 38**
**D-67063 Ludwigshafen (DE)**

(72) Erfinder : **Schroeder, Wolfgang, Dr.**
**Seebacher Strasse 51**
**W-6702 Bad Duerkheim (DE)**
Erfinder : **Ruider, Guenther, Dr.**
**In der Dreispitz 19**
**W-6706 Wachenheim (DE)**

**EP 0 446 783 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein verbessertes Verfahren zur Herstellung von N-arylsubstituierten 2-Aminoalkyl-2-hydroxyalkylaminen und N-arylsubstituierten Piperazinen der allgemeinen Formeln Ia bzw. Ib

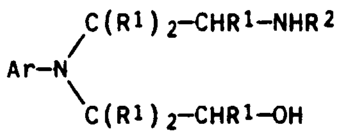

Ia

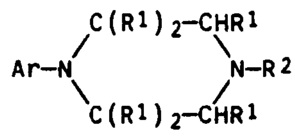

Ib

in denen Ar einen Arylrest, die Reste $R^1$, die gleich oder verschieden sein können, Wasserstoff oder Methylgruppen und der Rest $R^2$ Wasserstoff oder eine Alkylgruppe bedeuten, durch Umsetzung eines N,N-Di-(2-hydroxyalkyl)-N-aryl-amins der allgemeinen Formel II

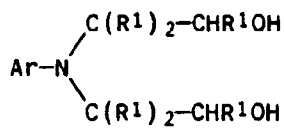

II

mit Ammoniak bzw. einem primären Amin der allgemeinen Formel III

$H_2N\text{-}R^2$ III

bei erhöhter Temperatur und unter erhöhtem Druck in Gegenwart von Wasserstoff und eines Katalysators.

Die Synthese von Piperazinen durch Umsetzung von Diethanolaminen mit Ammoniak oder primären Aminen an Hydrierkontakten ist allgemein bekannt (s. Houben-Weyl, Bd. XI/1, S. 126 ff, Thieme-Verlag, Stuttgart, 1957). Speziell in der DE-A-36 05 005 wird die Synthese von N-Methylpiperazin aus Diethanolamin und Methylamin in Gegenwart von Wasserstoff und kupferhaltigen Katalysatoren beschrieben. Andere N-substituierte Piperazine, die beispielsweise in der DE-A-26 24 042 beschrieben sind, waren bisher nur in mäßigen Ausbeuten erhältlich.

Die Verbindungen Ib wurden bisher nur in niedrigen Ausbeuten durch reduktive Ammonolyse (s. Yuki Gosei Kagaku Kyokai Ski 17 (1959) 17) oder durch Kondensationsreaktion von Aminoalkylestern mit Imiden (s. Nippon Kagaku Zasshi 89 (1968) 408) dargestellt.

Der Erfindung lag daher ein Verfahren zur Herstellung von N-arylsubstituierten 2-Aminoalkyl-2-hydroxyalkylaminen Ia und N-arylsubstituierten Piperazinen Ib als Aufgabe zugrunde, durch welches die Verbindungen Ia und Ib in höheren Ausbeuten und höherer Selektivität zugänglich gemacht werden als bisher.

Demgemäß wurde ein Verfahren zur Herstellung von N-arylsubstituierten 2-Aminoalkyl-2-hydroxyalkylaminen und N-arylsubstituierten Piperazinen der allgemeinen Formeln Ia bzw. Ib

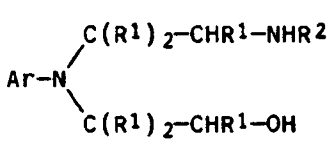

Ia

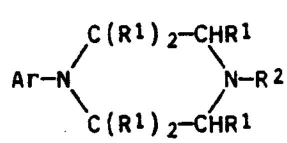

Ib

in denen Ar einen Arylrest, die Reste $R^1$, die gleich oder verschieden sein können, Wasserstoff oder Methylgruppen und der Rest $R^2$ Wasserstoff oder eine Alkylgruppe bedeuten, durch Umsetzung eines N,N-Di-(2-hydroxyalkyl)-N-aryl-amins der allgemeinen Formel II

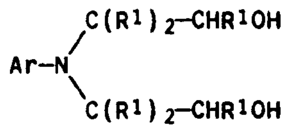

II

mit Ammoniak bzw. einem primären Amin der allgemeinen Formel III

$$H_2N\text{-}R^2 \qquad III$$

bei erhöhter Temperatur und unter erhöhtem Druck in Gegenwart von Wasserstoff und eines Katalysators gefunden, welches dadurch gekennzeichnet ist, daß man hierzu einen Trägerkatalysator oder einen Katalysator ohne Träger verwendet, dessen aktive Masse überwiegende Mengen an Kupfer und/oder Nickel und/oder Cobalt in metallischer oder oxidischer Form enthält.

Nach den bisherigen Beobachtungen hat die Art der Reste Ar, $R^1$ und $R^2$ prinzipiell keinen Einfluß auf das erfindungsgemäße Verfahren, sofern sie keine unter den Reaktionsbedingungen reaktive Substituenten tragen.

Als Reste Ar kommen unsubstituiertes oder ein-, zwei- oder dreifach mit Hydroxyl- oder $C_1$-$C_4$-Alkylgruppen substituiertes 1- oder 2-Naphthyl und insbesondere Phenyl in Betracht wie 2-, 3- und 4-Hydroxyphenyl, 2-, 3- und 4-Methylphenyl, 2, 4, 6-Trimethylphenyl, 4-Isopropylphenyl, 4-tert.-Butylphenyl, 2-Methylnaphth-1-yl und 4-Methylnaphth-1-yl.

Die Reste $R^1$, die gleich oder verschieden sein können, stehen für Wasserstoff oder Methyl, wobei Wasserstoff als Substituent bevorzugt wird.

Als Reste $R^2$ kommen in Betracht: Wasserstoff, $C_1$-$C_8$-Alkyl, vor allem Methyl, Ethyl, i-Propyl und tert.-Butyl, $C_3$-$C_8$-Cycloalkyl wie Cyclopropyl, Cyclopentyl und bevorzugt Cyclohexyl.

Die aktive Masse der erfindungsgemäß zu verwendenden Katalysatoren besteht entweder aus einem Kupferoxid, einem Cobaltoxid oder Nickeloxid oder Gemischen dieser Oxide allein, oder sie enthält sie in überwiegenden Anteilen, also zu mindestens 50, vorzugsweise 90 Gew.-% neben weiteren Bestandteilen, beispielsweise Trimangantetroxid, Molybdäntrioxid, Mangan(II)oxid, Phosphorsäure, sowie metallische oder als Oxide vorliegende Verbindungen von Chrom, Ruthenium, Silber, Palladium.

Als Trägermaterialien eignen sich vor allem unter den Reaktionsbedingungen nicht-reduzierbare Oxide wie Aluminiumoxid, Siliciumoxid, Titandioxid, Zirkonoxid und Aluminiumsilikate sowie Gemische dieser Materialien.

Die Trägerkatalysatoren sind in an sich bekannter Weise erhältlich, z.B. dadurch, daß man das Trägermaterial in einer wäßrigen Lösung der Salze der den aktiven Oxiden zugrundeliegenden Metalle, z.B. den Nitraten oder Acetaten, suspendiert und die Suspension mit Alkalimetall-Carbonaten oder -hydrogencarbonaten versetzt, wobei die Carbonate bzw. Hydrogencarbonate der Metalle der aktiven Masse auf die Trägerteilchen ausgefällt werden. Danach trennt man den Feststoff ab, trocknet ihn und erhitzt ihn im Luftstrom auf 350-600°C, wobei die Carbonate bzw. Hydrogencarbonate in die Oxide übergehen.

Die so erhaltene Masse kann nach anschließendem Mahlen und Aussieben auf eine geeignete Korngrößenverteilung, z.B. auf Durchmesser von 50 bis 300 $\mu$ verwendet und/oder durch bekannte Methoden zu Ringen, Tabletten oder Kugeln verpreßt und als fest angeordneter Katalysator eingesetzt werden.

Während oder vorzugsweise vor der Reaktion werden die oxidischen Massen durch Reduzieren mit Wasserstoff in die aktive Form überführt. Bei der Vorbehandlung wird ein Gemisch aus 5-20 Vol.-% Wasserstoff und 80-95 Vol.-% Stickstoff zunächst 4 bis 10 Stunden lang bei 120-160°C über den Katalysator geleitet, wonach die Temperatur im Laufe von 3 bis 5 Stunden auf 160-200°C erhöht und die Reduktion 3 bis 5 weitere Stunden fortgesetzt wird. Schließlich wird der Stickstoff durch Wasserstoff ersetzt, und die Temperatur wird auf 250-300°C erhöht. Nach weiteren 6 bis 10 Stunden ist die Reduktion beendet.

Die Menge des Katalysators beträgt zweckmäßigerweise 1 bis 100 g aktive Masse pro Mol der eingesetzten Dihydroxyverbindung II.

Vorzugsweise nimmt man die Umsetzung in der Weise vor, daß man den Katalysator in der die Reaktionspartner enthaltenden flüssigen Phase suspendiert oder die Reaktanden in flüssiger Phase über ein Katalysatorfestbett leitet.

Die Ausgangsverbindungen setzt man vorzugsweise im Molverhältnis Ammoniak bzw. Amin III zu Dihydroxyalkylamin II von 1:1 bis 150:1 ein.

Verfahrenstechnisch ist es vorteilhaft, die festen und flüssigen Ausgangsverbindungen II und III in unter den Reaktionsbedingungen inerten Lösungsmitteln aufzunehmen. Hierzu eignen sich vor allem Tetrahydrofuran, Dioxan, Ethylenglykoldimethylether oder N-Methylpyrrolidon in Mengen von 0,5 bis 2 kg pro kg der Ausgangsstoffe II und III.

Unter Normalbedingungen gasförmige Ausgangsverbindungen III speist man vorzugsweise ohne Lösungsmittel unter Druck in flüssiger Phase in den Reaktor ein.

Für die Selektivität und Aktivität des Katalysators ist es essentiell, die Reaktion in Gegenwart von Wasserstoff durchzuführen. Der Gesamtdruck, gebildet aus den Partialdrucken der Ausgangsverbindungen, des Lösungsmittels (falls vorhanden) und des Wasserstoffs, liegt vorzugsweise im Bereich von 40 bis 400 bar, bevorzugt von 100 bis 300 bar, wobei der Wasserstoffpartialdruck 80 bis 90 % davon beträgt.

Der Temperaturbereich zur Bildung der Produkte Ia und Ib liegt zwischen 100 und 300°C, bevorzugt zwischen 160 und 245°C.

Das Produktverhältnis Ia zu Ib läßt sich vorzugsweise durch Wahl der Temperatur und des Katalysatorsystems steuern. So erhält man bevorzugt die Piperazine Ib bei einer vorgegebenen Katalysatorzusammensetzung in der Regel bei Temperaturen, die um 20 bis 40°C höher liegen als diejenigen, die bei der Synthese der entsprechenden Amine Ia verwendet werden.

Fehlen andererseits die unter den Bedingungen nicht reduzierbaren als Trägermaterial eingesetzten Oxide, so werden unabhängig von der Temperaturwahl im genannten Bereich bevorzugt die 2-Aminoalkyl-2-hydroxyalkylamine Ia gebildet (s. Beispiele).

Das Verfahren kann kontinuierlich oder diskontinuierlich ausgeführt werden, wobei im diskontinuierlichen Betrieb die Reaktionsdauer von 0,02 bis 1 h beträgt.

Die Reinigung des Rohproduktes führt man in üblicher Weise durch bekannte Verfahren wie fraktionierende Destillation oder Kristallisation durch.

Die Verbindungen I sind wichtige Ausgangsstoffe für die Herstellung von psychotrop wirksamen Arzneimitteln wie Tranquilizern, Neuroleptika oder Analgetika (Coll. Czech. Chem. Comm. 40 (1975) 1204, 1612, EP-B-190 472) sowie von Kosmetika und Fotochemikalien. Sie finden auch Verwendung in der Nahrungs- und Futtermittelindustrie (DE-A-26 24 042). Einige der Verbindungen Ia werden auch in der Analytik zur quantitativen $H_2O_2$-Bestimmung eingesetzt (JP-A-60241899).

Beispiele

A Zusammensetzung der verwendeten Katalysatoren

Tabelle 1: Katalysatorzusammensetzungen

| | Katalysator [Gew.-%] | | | | | |
|---|---|---|---|---|---|---|
| | A | B | C | D | E | F |
| **aktive Masse** | | | | | | |
| Cobalt(II)oxid | 10 | 10 | 16 | - | - | 66 |
| Nickel(II)oxid | 10 | 10 | - | 26 | - | - |
| Kupfer(II)oxid | 4 | 4 | - | 9 | - | 20 |
| Kupfer | - | - | - | - | 44 | - |
| Trimangantetroxid | - | - | - | 2,5 | - | 7 |
| Molybdäntrioxid | - | - | - | - | - | 4 |
| Phosphorsäure | - | - | - | - | - | 3 |
| **Trägermaterial** | | | | | | |
| Aluminiumoxid | - | - | 83 | - | 56 | - |
| Siliciumdioxid | - | 76 | - | 62,5 | - | - |
| Zirkondioxid | 76 | - | - | - | - | - |
| Mangan(II)oxid | - | - | 1 | - | - | - |

B Umsetzungen

Die Versuche wurden in kontinuierlicher Verfahrensweise durchgeführt. Dazu wurde ein Rohrreaktor von

5 l Füllvolumen zu 60 % mit Katalysator befüllt. Der Katalysator bestand aus den unter A beschriebenen Mischungen, die zu zylindrischen Tabletten (Höhe = Durchmesser = 4 mm) verpreßt waren. Der mit Wasserstoff durchspülte Reaktor wurde anschließend aufgeheizt, bis die Reaktionstemperatur erreicht war, dann wurden die Ausgangsstoffe in den Reaktor eingespeist. Der Reaktionsdruck betrug 250 bar und wurde durch Aufpressen von Wasserstoff erreicht. Nach Verlassen des Reaktors wurde das Reaktionsgemisch abgekühlt, entspannt und fraktionierend destilliert. Eine Ausnahme bildete dabei die Reinigung des Produktes aus Versuch 7 (4-Hydroxyphenylpiperazin), die nicht durch Destillation, sondern durch Kristallisation in Tetrahydrofuran erfolgte. Die Produkte wurden stets in einer Reinheit von über 99 % erhalten. Die Bestimmung der Ausbeuten und die Analyse der Produkte erfolgte mit üblichen Methoden wie GC und NMR.

In den Versuchen 1 bis 6 und 9 wurde das Dihydroxyalkylamin II in Mengen von 1,5 mol/h als 1,5 molare Tetrahydrofuranlösung gleichzeitig mit flüssigem Ammoniak (120 mol/h) in den Reaktor eingespeist.

In Versuch 7 wurden 1,2 mol/h Dihydroxyalkylamin II als 1,2 molare Lösung in N-Methylpyrrolidon und 120 mol/h flüssiger Ammoniak eingesetzt.

In Versuch 8 wurden 4,9 mol/h Dihydroxyalkylamin II und 15,1 mol/h Isopropylamin in einer Lösung bestehend aus 4,9 mol Dihydroxyalkylamin II, 15, 1 mol Isopropylamin pro l Tetrahydrofuran eingesetzt.

In Tabelle 2 sind weitere Einzelheiten zu den Versuchen sowie die Ergebnisse aufgeführt.

Tabelle 2: Versuchsergebnisse

| Beispiel Nr. | Katalysator | $ArN(CH_2CH_2OH)_2$ II Ar | $H_2NR^2$ $R^2$ | Temperatur [°C] | Umsatz [%] | Produkt | Ausbeute [%] |
|---|---|---|---|---|---|---|---|
| 1 | A | Phenyl | H | 180 | 100 | N-Phenylpiperazin Ib | 75 |
| 2 | B | Phenyl | H | 180 | 100 | N-Phenylpiperazin Ib | 75 |
| 3 | C | Phenyl | H | 190 | 100 | N-Phenylpiperazin Ib | 70 |
| 4 | D | Phenyl | H | 180 | 100 | N-Phenylpiperazin Ib<br>N-(2-aminoethyl)-N-(2-hydroxyethyl)anilin Ia | 8<br>60 |
| 5 | D | Phenyl | H | 220 | 100 | N-Phenylpiperazin Ib<br>N-(2-aminoethyl)-N-(2-hydroxyethyl)anilin Ia | 55<br>1 |
| 6 | A | 2-Tolyl | H | 180 | 100 | N-o-Tolylpiperazin Ib | 70 |
| 7 1) | A | 4-Hydroxyphenyl | H | 180 | 100 | N-(p-Hydroxyphenyl)-piperazin Ib | 65 |
| 8 | E | Phenyl | iPr | 200 | 98 | N-Isopropyl-N'-phenyl-piperazin Ib | 65 |
| 9 | F | Phenyl | H | 180 | 60 | N-Phenylpiperazin Ib<br>N-(2-aminoethyl)-N-(2-hydroxyethyl)anilin Ia | 20<br>75 |

1) in N-Methylpyrrolidon

**Patentansprüche**

1. Verfahren zur Herstellung von N-arylsubstituierten 2-Aminoalkyl-2-hydroxyalkylaminen und N-arylsubstituierten Piperazinen der allgemeinen Formeln Ia bzw. Ib

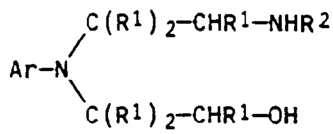

Ia

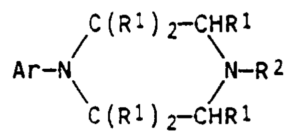

Ib

in denen Ar einen Arylrest, die Reste $R^1$, die gleich oder verschieden sein können, Wasserstoff oder Methylgruppen und der Rest $R^2$ Wasserstoff oder eine Alkylgruppe bedeuten, durch Umsetzung eines N,N-Di-(2-hydroxyalkyl)-N-aryl-amins der allgemeinen Formel II

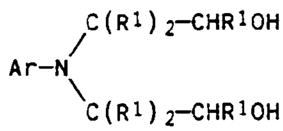

II

mit Ammoniak bzw. einem primären Amin der allgemeinen Formel III

$H_2N-R^2$ III

bei erhöhter Temperatur und unter erhöhtem Druck in Gegenwart von Wasserstoff und eines Katalysators, dadurch gekennzeichnet, daß man hierzu einen Trägerkatalysator oder einen Katalysator chne Träger verwendet, dessen aktive Masse überwiegende Mengen an Kupfer und/oder Nickel und/oder Cobalt in in metallischer oder oxidischer Form enthält.

2. Verfahren nach Anspruch 1, dadurch gekennzeichnet, daß das Trägermaterial des Katalysators aus Aluminiumoxid, Siliciumdioxid, Zirkondioxid, Titandioxid, Aluminiumsilikaten oder Gemischen davon besteht.

**Claims**

1. A process for preparing N-aryl-substituted 2-aminoalkyl-2-hydroxyalkylamines and N-aryl-substituted piperazines of the formulae Ia and Ib respectively

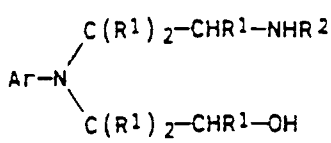

Ia

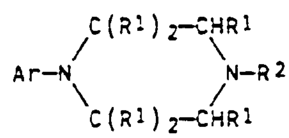

Ib

where Ar is aryl, the $R^1$ radicals can be identical or different and are hydrogen or methyl, and $R^2$ is hydrogen or alkyl, by reacting an N,N-di(2-hydroxyalkyl)-N-arylamine of the formula II

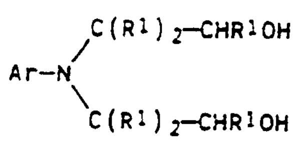

II

with ammonia or a primary amine of the formula III

$H_2N\text{-}R^2$ III

at elevated temperature and under elevated pressure in the presence of hydrogen and of a catalyst, which comprises using for this a supported or unsupported catalyst whose active mass contains predominant amounts of copper and/or nickel and/or cobalt in the form of the metal or an oxide.

2. A process as claimed in claim 1, wherein the catalyst carrier material is composed of alumina, silica, zirconia, titania, aluminum silicates or mixtures thereof.

## Revendications

1. Procédé de préparation de 2-amino-alkyl-2-hydroxyalkylamines à substitution N-arylique et de pipérazines à substitution N-arylique répondant aux formules Ia et respectivement Ib ci-dessous

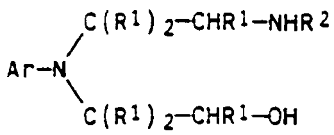

Ia

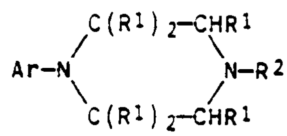

Ib

dans lesquelles Ar représente un radical aryle, les symboles $R^1$, qui peuvent avoir des significations identiques ou différentes, représentent des atomes d'hydrogène ou des radicaux méthyle et le symbole $R^2$ représente un atome d'hydrogène ou un radical alkyle, par la réaction d'une N,N-di-(2-hydroxyalkyl)-N-arylamine de la formule II

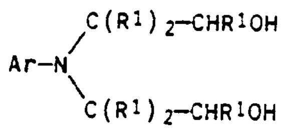

II

avec de l'ammoniac ou une amine primaire de la formule générale III

$H_2N\text{-}R^2$ III

à température élevée et sous pression élevée en présence d'hydrogène et d'un catalyseur, caractérisé en ce que l'on utilise à cette fin un catalyseur sur support ou un catalyseur sans support, dont la masse active contient des quantités prépondérantes de cuivre et/ou de nickel et/ou de cobalt sous forme métallique ou oxydée.

2. Procédé suivant la revendication 1, caractérisé en ce que la matière constitutive du support du catalyseur est constituée d'oxyde d'aluminium, de dioxyde de silicium, de dioxyde de zirconium, de dioxyde de titane, de silicates d'aluminium ou de mélanges de ces composés.